# EUROPEAN PATENT SPECIFICATION

(11) **EP 3 701 877 B1**
(45) Date of publication and mention of the grant of the patent: **22.06.2022**
(21) Application number: 18870819.2
(22) Date of filing: 23.10.2018
(51) Int. Cl.: A61B 8/14, A61B 8/00, A61B 8/08, A61B 8/15, G01S 15/89

(54) **ULTRASOUND DIAGNOSTIC SYSTEM AND ULTRASOUND DIAGNOSTIC METHOD**
ULTRASCHALLDIAGNOSESYSTEM UND ULTRASCHALLDIAGNOSEVERFAHREN
SYSTÈME ET PROCÉDÉ DE DIAGNOSTIC PAR ULTRASONS

(30) Priority: 24.10.2017 JP 2017205343
(43) Date of publication of application: 02.09.2020
(73) Proprietor: Lily Medtech Inc., Tokyo 113-0033 (JP); The University of Tokyo, Tokyo 113-0033 (JP)
(72) Inventor: AZUMA, Takashi, Tokyo 113-0033 (JP); TANG, Tianhan, Tokyo 113-0033 (JP); SAKUMA, Ichiro, Tokyo 113-0033 (JP); NAKAMURA, Hirofumi, Tokyo 113-0033 (JP)
(74) Representative: Hoffmann Eitle
(86) International application number: PCT/JP2018/039356
(87) International publication number: WO 2019/082892

(56) References cited:
- JP-A- 2005 342 140
- JP-A- 2009 240 667
- US-A1- 2011 146 371
- US-A1- 2013 204 137
- LI C ET AL: "In vivo Breast Sound-Speed Imaging with Ultrasound Tomography", ULTRASOUND IN MEDICINE AND BIOLOGY, NEW YORK, NY, US, vol. 35, no. 10, 1 October 2009 (2009-10-01), pages 1615-1628, XP026641057, ISSN: 0301-5629, DOI: 10.1016/J.ULTRASMEDBIO.2009.05.011 [retrieved on 2009-08-03]

## Description

### Technical Field

The present invention relates to an ultrasound diagnostic system and an ultrasound diagnostic method in which ultrasound irradiation is performed and a tomographic image of a test object is generated.

### Background Art

A noninvasive diagnostic system using ultrasound is widely used in the medical field as a technology for making a diagnosis based on information regarding the inside of a test object since there is no need to perform surgery in which a direct incision is made to carry out an observation in a living body.

In ultrasound computed tomography (CT), which is a technique for making a diagnosis using ultrasound, a test object is irradiated with ultrasound and a tomographic image of the test object is generated using reflected ultrasound or transmitted ultrasound. A recent study shows that ultrasound CT is useful in detecting breast cancer. In ultrasound CT, for example, a ring array transducer obtained by arranging, in a ring shape, many elements that emit and receive ultrasound is used to generate tomographic images.

In the synthetic aperture method, which is one of conventional tomographic image generation methods, first, ultrasound is emitted from one element, an echo signal is received by all the elements, and two-dimensional data (frame data) is generated in which a first axis represents receiving element number and a second axis represents echo signal arrival time. Frame data sets, the number of which is equal to the number of elements of the ring array transducer, are generated by changing in order the element that emits ultrasound.

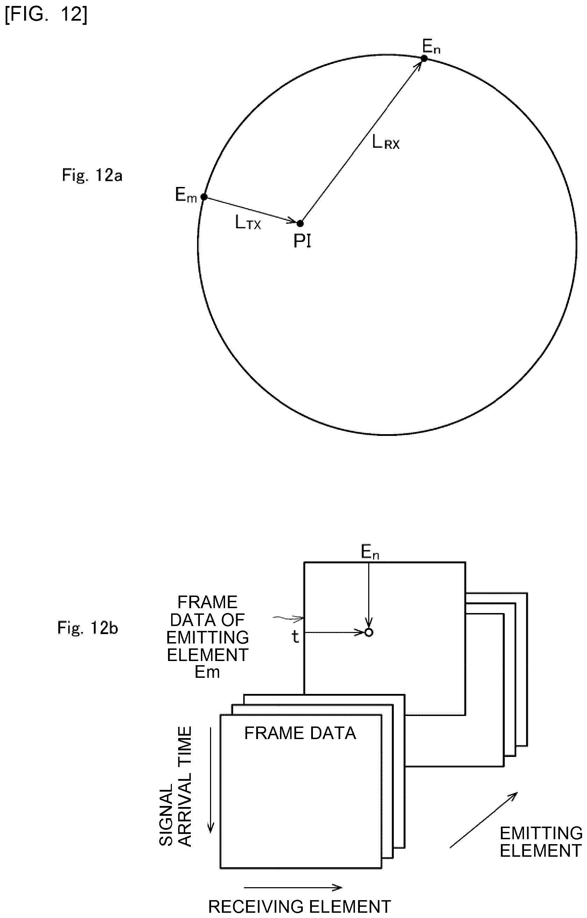

As illustrated in Fig. 12a, an echo signal arrival time t = (L_{TX} + L_{RX})/c can be obtained from a distance L_{TX} from an emitting element Em to a point of interest PI corresponding to a pixel of a tomographic image, a distance L_{RX} from this point of interest PI to a receiving element En, and a sound speed c. As illustrated in Fig. 12b, in the frame data of the emitting element Em, echo data at the receiving element En and at the time t correspond to the point of interest PI.

One frame data set includes, for each receiving element, echo data corresponding to the point of interest PI. In a case where the ring array transducer is constituted by N elements, the number of receiving elements is N, and thus one frame data set includes N pieces of echo data corresponding to the point of interest PI. There are N frame data sets, and thus the brightness of one pixel corresponding to the point of interest PI is a composition of N × N pieces of echo data. An image is generated by calculating the brightness of each pixel in this manner.

As described above, hitherto, receiving of an echo signal of ultrasound emitted from one element using all the elements is repeatedly performed a number of times equal to the number of elements, and thus the number of times ultrasound is emitted is large and it takes time to perform measurement. In addition, since a large amount of data is acquired, it takes time to transfer the data to a calculating machine.

PTL 1: International Publication No. 2017/051903

### Summary of Invention

Document US2013/204137 describes a method and system for denoising acoustic travel times and imaging a volume of tissue comprising receiving a dataset representative of acoustic waveforms originating from an array of ultrasound emitters and received with an array of ultrasound receivers. According to this document for each ultrasound emitter in the array of ultrasound emitters, an empirical relative travel time matrix is formed, from the dataset, including a set of relative empirical travel times, each relative empirical travel time corresponding to a pair of ultrasound receivers receiving an acoustic waveform. A denoised empirical relative travel time matrix is generated, a set of denoised absolute travel times from the denoised empirical relative travel time matrix is extracted and an image of the volume of tissue is rendered based on an acoustomechanical parameter and the set of denoised absolute travel times corresponding to each ultrasound emitter in the array of ultrasound emitters.

The present invention has been made in light of the conventional circumstances described above, and an object of the present invention is to provide an ultrasound diagnostic system and an ultrasound diagnostic method that make it possible to shorten the time required to measure a test object and to transfer data.

The invention is defined in the independent claims 1 and 9. An ultrasound diagnostic system according to the present invention includes a plurality of elements that are arranged around a test object and perform at least either emission or reception of ultrasound, a control unit that controls the plurality of elements such that any one of the plurality of elements emits ultrasound and all or some of the plurality of elements receive scattered waves caused by the test object scattering the ultrasound, a data collection unit that collects measurement data, which are data obtained from the elements that have received the scattered waves, a calculation unit that calculates, for division regions into which an imaging region including all or a portion of the test object is divided, a scattered sound pressure intensity of each division region, which is the intensity of sound pressure of the scattered waves in the division region, on the basis of a first factor and a second factor of the division region, the first factor being constituted by arrival times which are each a period from emission to reception of ultrasound that is emitted from a predetermined element among the plurality of elements, scattered by the test object in the division region, and received by a corresponding one of all or some of the plurality of elements, the second factor being constituted by the measurement data, and an image generation unit that generates a scattering image, which is an image obtained by converting the scattered sound pressure intensity of each division region into a pixel value.

According to an aspect of the present invention, the division regions are regions obtained by dividing the imaging region in a grid-like manner, the first factor is an inverse matrix of a matrix constituted by the arrival times, the second factor is a vector constituted by the measurement data, and the calculation unit calculates the scattered sound pressure intensity from the product of the first factor and the second factor.

According to an aspect of the present invention, the product of the number of vertical regions and the number of horizontal regions, the vertical and horizontal regions constituting the division regions and being obtained by performing division in a grid-like manner, and the product of the number of receiving elements and the number of data samples in a time-axis direction in collection of the measurement data respectively match the number of columns and the number of rows of the matrix.

According to an aspect of the present invention, the control unit performs control such that a second element emits ultrasound after a first element emits ultrasound, the data collection unit collects first measurement data from an element that has received a scattered wave corresponding to the ultrasound emitted by the first element and collects second measurement data from an element that has received a scattered wave corresponding to the ultrasound emitted by the second element, and the calculation unit calculates a first scattered sound pressure intensity from the product of a first inverse matrix obtained in a case where the first element is treated as an ultrasound emitting element and a vector obtained by arranging the first measurement data, calculates a second scattered sound pressure intensity from the product of a second inverse matrix obtained in a case where the second element is treated as an ultrasound emitting element and a vector obtained by arranging the second measurement data, and combines the first scattered sound pressure intensity and the second scattered sound pressure intensity.

According to an aspect of the present invention, a rank of the matrix is equal to the product of the number of vertical regions and the number of horizontal regions, the vertical and horizontal regions being regions obtained by performing division in the grid-like manner.

According to an aspect of the present invention, the arrival times are calculated on the basis of the fact that there is a difference in the sound speed of the ultrasound inside a breast and the sound speed of the ultrasound outside the breast.

According to an aspect of the present invention, when seen from the test object, the receiving elements are arranged on a side where the emitting element is arranged.

According to an aspect of the present invention, the scattering image is generated every predetermined time and a portion of the generated scattering image where a change in pixel value is greater than or equal to a predetermined value is extracted.

An ultrasound diagnostic method according to the present invention includes a step of emitting ultrasound from any one of a plurality of elements arranged around a test object and receiving, using all or some of the plurality of elements, scattered waves caused by the test object scattering the ultrasound, a step of collecting measurement data, which are data obtained from the elements that have received the scattered waves, a step of calculating, for division regions into which an imaging region including all or a portion of the test object is divided, a scattered sound pressure intensity of each division region, which is the intensity of sound pressure of the scattered waves in the division region, on the basis of a first factor and a second factor of the division region, the first factor being constituted by arrival times which are each a period from emission to reception of ultrasound that is emitted from a predetermined element among the plurality of elements, scattered by the test object in the division region, and received by a corresponding one of all or some of the plurality of elements, the second factor being constituted by the measurement data, and a step of generating a scattering image, which is an image obtained by converting the scattered sound pressure intensity of each division region into a pixel value.

### Advantageous Effects of Invention

According to the present invention, the time required to measure a test object and to transfer data can be shortened.

### Brief Description of Drawings

[Fig. 1] Fig. 1 is a schematic configuration diagram of an ultrasound diagnostic system according to an embodiment of the present invention.
[Fig. 2] Fig. 2 is a section view taken along line A-A of Fig. 1.
[Fig. 3] Fig. 3 is a functional block diagram of a calculation device.
[Fig. 4] Fig. 4 is a diagram illustrating an example of received scattered waves.
[Fig. 5] Fig. 5 is a diagram illustrating an example of received scattered waves.
[Fig. 6] Fig. 6 is a diagram illustrating an example of pixel division of a region of interest.
[Fig. 7] Figs. 7a to 7d are diagrams for describing a measurement matrix generation method.
[Fig. 8] Fig. 8a is a diagram illustrating a phantom, and Figs. 8b to 8f are diagrams illustrating simulation results obtained when an image generation method of a comparative example was applied.
[Fig. 9] Fig. 9a is a diagram illustrating a phantom, and Figs. 9b to 9f are diagrams illustrating simulation results obtained when the image generation method of the comparative example was applied.
[Fig. 10] Figs. 10a and 10d are diagrams illustrating a phantom, and Figs. 10b and 10e are diagrams illustrating simulation results obtained when an image generation method according to an embodiment was applied.
[Fig. 11] Fig. 11a is a diagram illustrating an example of a receiving aperture restriction, and Fig. 11b is a diagram illustrating an example of a transmitted wave propagation time and a scattered wave propagation time.
[Fig. 12] Figs. 12a and 12b are diagrams for describing a data acquisition method using the conventional synthetic aperture method.
[Fig. 13] Figs. 13a and 13b are diagrams illustrating an evaluation model.
[Fig. 14] Figs. 14a and 14b are diagrams illustrating reconstructed images according to the embodiment.
[Fig. 15] Figs. 15a and 15b are diagrams illustrating reconstructed images according to the synthetic aperture method.
[Fig. 16] Fig. 16a is a graph illustrating signal-to-noise ratio analysis results, and Fig. 16b is a graph illustrating resolution analysis results.

### Description of Embodiments

In the following, the present invention will be described in more detail with reference to the drawings. An ultrasound diagnostic system according to an embodiment of the present invention irradiates a test object such as a human body with ultrasound and generates a scattering image (a map of scattered sound pressure intensities) using received echo signals. Doctors can make a diagnosis of a lesion such as a malignant tumor by checking the generated scattering image.

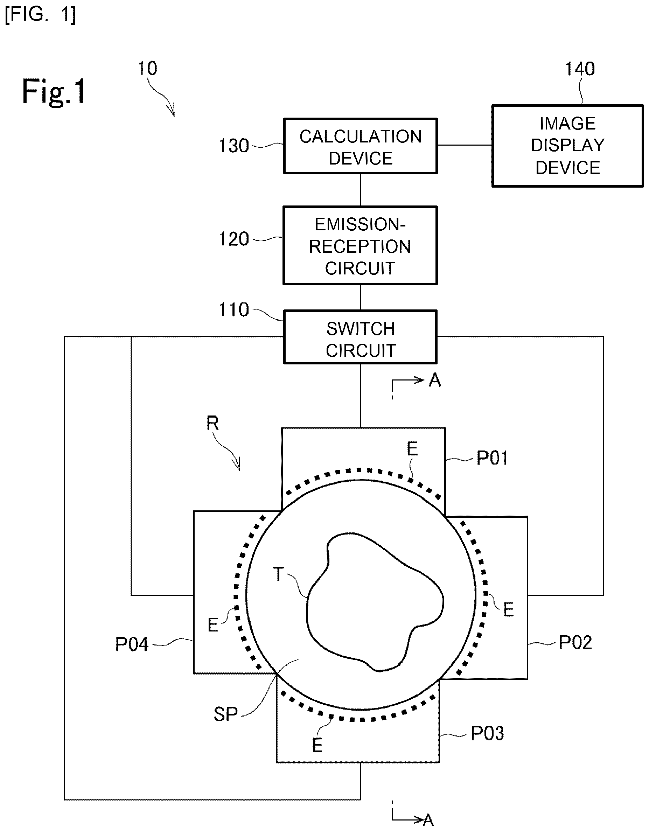

As illustrated in Fig. 1, an ultrasound diagnostic system 10 according to the present embodiment includes a ring array R, a switch circuit 110, an emission-reception circuit 120, a calculation device 130, and an image display device 140.

The ring array R is a ring-shaped transducer constituted by a combination of a plurality of transducers and having preferably a diameter of 80 to 500 mm and more preferably a diameter of 100 to 300 mm. The ring array R may have a variable diameter. In the present embodiment, as an example, a ring-shaped transducer obtained by combining four concave transducers P01 to P04 is used.

For example, in a case where the concave transducers P01 to P04 each have 256 rectangular piezoelectric elements E (hereinafter also simply referred to as "elements E"), the ring array R is constituted by 1024 elements E. The number of elements E provided at the concave transducers P01 to P04 is not limited to a specific number, and is preferably between 1 and 1000 and more preferably between 100 and 500.

Each element E has the function of converting an electrical signal into an ultrasonic signal and converting an ultrasonic signal into an electrical signal. The element E emits ultrasound to a test object T, receives scattered waves that are waves scattered (reflected) by the test object T (forward scattered waves, side scattered waves, and back scattered waves), and forms an electrical signal as measurement data.

In the present embodiment, each element E is described as an element having the function of both emitting and receiving ultrasound; however, the element E is not limited to this. For example, emitting elements or receiving elements may be used, which have either one of the function of emitting ultrasound and the function of receiving ultrasound, and a plurality of emitting elements and a plurality of receiving elements may be arranged in a ring shape. In addition, the ring array R may be constituted by the element (or elements) having the function of both emitting and receiving ultrasound, the emitting element (or elements), and the receiving element (or elements) in a mixed manner.

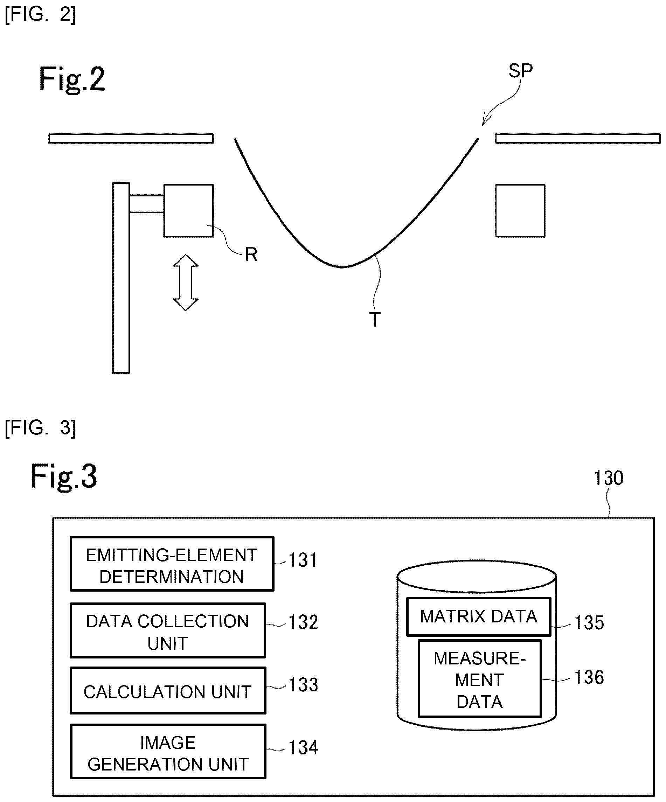

Fig. 2 is a section view taken along line A-A of Fig. 1. For example, the ring array R is installed under a bed having an opening such that the opening of the bed is superposed with an insertion portion SP. A test subject inserts a site of his or her body to be imaged (the test object T) into the insertion portion SP from the opening of the bed.

The insertion portion SP, into which the test object T is inserted, is provided at the center of the ring array R. The plurality of elements E of the ring array R are provided at equal intervals along the ring around the insertion portion SP. Convex lenses called acoustic lenses are attached to the inner peripheral side surface of the ring array R. Such surface treatment added on the inner peripheral side of the ring array R can cause ultrasound emitted by each element E to converge within a plane including the ring array R.

In the present embodiment, the elements E are arranged in a ring shape and at equal intervals; however, the shape of the ring array R is not limited to a circular shape and may be, for example, an arbitrary polygonal shape such as a hexagon, a square, and a triangle, a shape having at least partially a curve or an arc, another arbitrary shape, or a portion of these shapes (for example, a semicircle or an arc). That is, the ring array R can be generalized as an array R. In addition, the elements E constituting the array R are preferably arranged intermittently around the test object T so as to cover 90 degrees or more; however, the arrangement of the elements E is not limited to these.

The ring array R is connected to the emission-reception circuit 120 with the switch circuit 110 interposed therebetween. The emission-reception circuit 120 (control unit) transmits a control signal (electrical signal) to the elements E of the ring array R and controls emission and reception of ultrasound. For example, the emission-reception circuit 120 sends, to the elements E, a command as to for example the frequency and magnitude of ultrasound to be emitted and the type of wave (such as a continuous wave or a pulse wave).

The switch circuit 110 is connected to each of the plurality of elements E of the ring array R, transfers a signal from the emission-reception circuit 120 to certain elements E among the plurality of elements E, and drives the elements E to emit-receive a signal. For example, by switching the elements E to which the control signal from the emission-reception circuit 120 is supplied, the switch circuit 110 causes one of the plurality of elements E to function as an emitting element that emits ultrasound and causes a plurality of elements E (for example, all the elements E) to receive scattered waves.

Measurement data may be collected by simultaneously driving all the elements E. Alternatively, the plurality of elements E of the ring array R are divided into some groups and measurement data may be collected in order on a group basis. By switching the group at a rate less than or equal to the order of a few microseconds to milliseconds, measurement data can be collected in almost real time.

The ring array R is installed so as to be movable up and down by, for example, a stepping motor. Data on the entirety of the test object T is collected by moving the ring array R up and down.

The calculation device 130 is constituted by, for example, a computer including a central processing unit (CPU), a memory unit (such as a random access memory (RAM), a read-only memory (ROM), and a hard disk), and a communication unit. The functions of, for example, an emitting-element determination unit 131, a data collection unit 132, a calculation unit 133, and an image generation unit 134 as illustrated in Fig. 3 are realized by executing a program stored in the memory unit, and a matrix data storage area 135 and a measurement data storage area 136 are reserved in the memory unit. Processing performed by each unit will be described later.

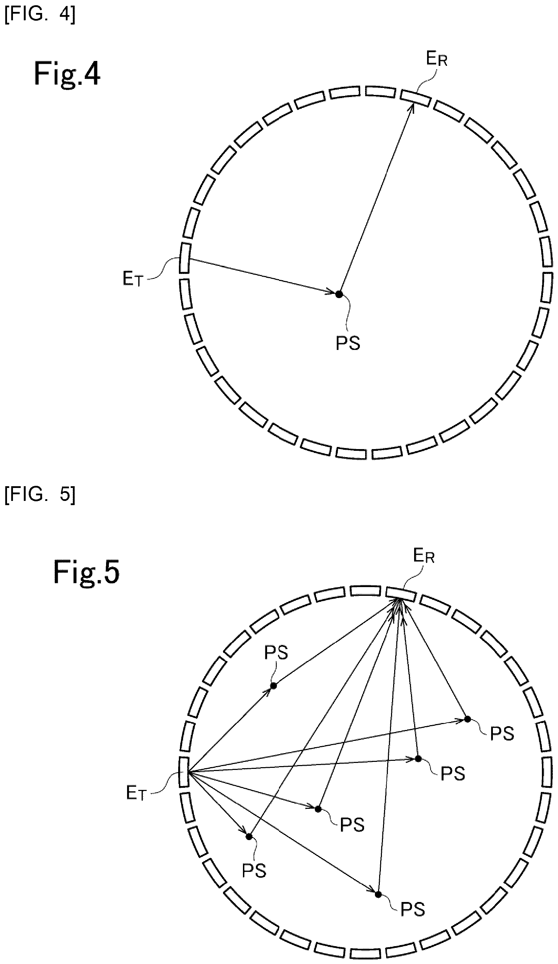

Next, a scattering image generation method according to the present embodiment will be described. As illustrated in Fig. 4, a case will be considered where, while focusing on one point scatterer PS (one point of the test object T), ultrasound emitted from a single emitting element E_{T} is scattered by this point scatterer PS and then received by a single receiving element E_{R}. In this case, measurement data from the receiving element E_{R} includes a unique pattern caused by the effect of this point scatterer PS.

In the present embodiment, assuming that the test object T is constituted by many point scatterers, a specific portion of the test object T will reflect and scatter ultrasound. In a case where many point scatterers PS exist as illustrated in Fig. 5, measurement data from the receiving element E_{R} includes a linear combination of unique patterns caused by the effect of the respective point scatterers PS. In the present embodiment, each unique pattern is isolated from the measurement data and is distinguished to calculate scattered sound pressure intensities, which are the intensities of sound pressure of scattered waves of the respective point scatterers PS.

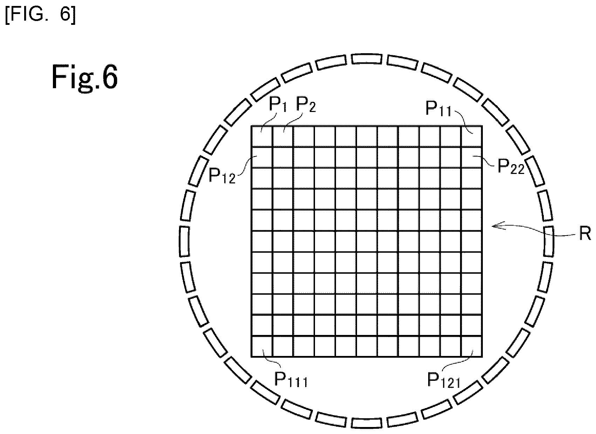

In the present embodiment, division regions are set that are obtained by dividing an imaging region including a front portion or a portion of the test object. Specifically, as illustrated in Fig. 6, an imaging region (region of interest (ROI)) R is divided in a grid-like manner, and a plurality of pixel regions are set. For example, the imaging region R is divided into M² (= M × M) pixel regions. Fig. 6 illustrates an example in which M = 11 and the imaging region R is divided into 121 pixel regions P₁ to P₁₂₁.

In the present embodiment, the scattered sound pressure intensity of scattered waves in each division region is calculated on the basis of signal arrival times and the measurement data. Each of the signal arrival times represents a period from emission to reception of ultrasound that is emitted from a predetermined element, scattered by a test object in the division region, and received by a corresponding one of the plurality of elements.

The scattered sound pressure intensity of each pixel region can be expanded to a one-dimensional vector x. In a case where there are M² pixel regions, there are M² × 1 vectors x. In a case where ultrasound is emitted from one specific emitting element E_{T}, N receiving elements E_{R} are used, and the number of samples per element is Nt, the measurement data can be expressed by Nt × N × 1 vectors y.

In this case, use of an appropriate measurement matrix G can result in mathematization as Gx = y. By using the inverse matrix G⁻¹ of G, x is obtained from x = G⁻¹y. That is, the scattered sound pressure intensity of each pixel region can be calculated from the inverse matrix G⁻¹ and the measurement data from the N receiving elements E_{R}.

Note that, other than a method using an inverse matrix, a method using a so-called pseudo-inverse matrix is also effective. This is a method in which, other than G⁻¹ satisfying G⁻¹G = E (E is an identity matrix), G' is used with which the sum of absolute values of traces becomes the smallest in G'G = E', where E' is a predetermined condition. G' can be obtained by using, for example, the method of least squares or the calculus of variations with constraints. This technique is advantageous in that post-processing noise caused by the divergence of the inverse matrix can be prevented from becoming larger. Likewise, a technique for calculating the inverse matrix H' of H = G + λE is also effective.

Next, the configuration of the measurement matrix G will be described. The measurement matrix G is constructed by fixing the emitting element E_{T}, regarding each pixel region as one point scatterer, and treating, as the i-th column vector, signal arrival times obtained in a case where a plurality of receiving elements receive ultrasound scattered by the i-th point scatterer.

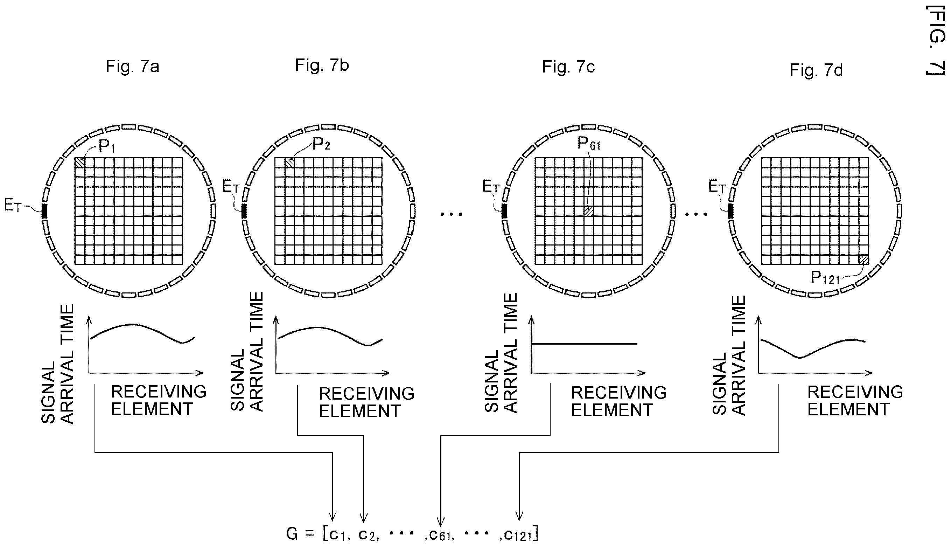

For example, as illustrated in Fig. 7a, a column vector c₁ in which signal arrival times are arranged in the order of arrangement of a plurality of receiving elements is the first column vector of the measurement matrix G, the signal arrival times each representing a period from emission to reception of ultrasound that is emitted from the emitting element E_{T}, scattered by a point scatterer positioned in the first pixel region P₁, and received by a corresponding one of the receiving elements (for example, all the receiving elements).

As illustrated in Fig. 7b, a column vector c₂ in which signal arrival times are arranged in the order of arrangement of the plurality of receiving elements is the second column vector of the measurement matrix G, the signal arrival times each representing a period from emission to reception of ultrasound that is emitted from the emitting element E_{T}, scattered by a point scatterer positioned in the second pixel region P₂, and received by a corresponding one of the receiving elements.

As illustrated in Fig. 7c, a column vector c₅₆ in which signal arrival times are arranged in the order of arrangement of the plurality of receiving elements is the 61st column vector of the measurement matrix G, the signal arrival times each representing a period from emission to reception of ultrasound that is emitted from the emitting element E_{T}, scattered by a point scatterer positioned in the 61st pixel region P₅₆, and received by a corresponding one of the receiving elements.

As illustrated in Fig. 7d, a column vector c₁₂₁ in which signal arrival times are arranged in the order of arrangement of the plurality of receiving elements is the 121st column vector of the measurement matrix G, the signal arrival times each representing a period from emission to reception of ultrasound that is emitted from the emitting element E_{T}, scattered by a point scatterer positioned in the 121st pixel region P₁₂₁, and received by a corresponding one of the receiving elements.

The measurement matrix G is constructed in this manner. The inverse matrix G⁻¹ of the measurement matrix G is calculated by an unillustrated calculating machine and is stored in the matrix data storage area 135.

The emitting-element determination unit 131 sends a command to the emission-reception circuit 120, so that ultrasound is emitted from the element E that is assumed to be the emitting element in this measurement matrix G.

The data collection unit 132 collects (including receives or acquires) measurement data (reception data), which are data obtained from the plurality of elements, via the switch circuit 110 and the emission-reception circuit 120. The measurement data is stored in the measurement data storage area 136.

The calculation unit 133 calculates scattered sound pressure intensity x of each pixel region from the product of the inverse matrix G⁻¹ stored in the matrix data storage area 135 and the vectors y constituted by the measurement data stored in the measurement data storage area 136.

The image generation unit 134 converts the scattered sound pressure intensity of each pixel region into a pixel value and generates a scattering image of the imaging region (a map of the scattered sound pressure intensities) by arranging the pixel values in a two-dimensional array. The generated scattering image is displayed on the image display device 140.

To make it possible to obtain the inverse matrix G⁻¹, the rank (rank) of the matrix G needs to be equal to the number of pixels M². The rank can also be called the number of different eigenvalues of this matrix. Conversely, the calculation device 130 is configured to determine, under the conditions that the rank of the matrix G = M² is satisfied, a correspondence between the number of pixels that is to be obtained and the number of receiving elements and acquire data under the conditions. In a case where the solution obtained by multiplying the pre-calculated G⁻¹ by acquired data y is undesirable (such as a case where a significant artifact exists), the calculation device 130 can be configured such that an image is reconstructed by multiplying G₂⁻¹, which is calculated from G₂ formed for the smaller number of pixels (M₂)² after the data acquisition, by data. (M₂ < M)

In this manner, according to the present embodiment, ultrasound is emitted from a single emitting element and a scattering image is generated by calculating the scattered sound pressure intensity of each pixel region of the imaging region (ROI) from the product of vectors obtained by arranging pieces of echo data received by the plurality of receiving elements and the inverse matrix G⁻¹ of the measurement matrix G, which is prepared in advance.

Thus, the time required to measure a test object can be made shorter than in the case of using the conventional synthetic aperture method. In the synthetic aperture method, the reception of echo signals of ultrasound, which is emitted from one element, using all the elements is repeatedly performed while switching the emitting element. In addition, the amount of data to be acquired can be reduced and the time required to transfer the data can be shortened.

The example in which only a single element serves as the emitting element has been described in the embodiment above and this is preferable in a case where the number of pixel regions is small and a case where there is significantly little noise. In a case where the number of pixel regions is large or a case where there is large noise, ultrasound is preferably emitted by switching in order between a plurality of emitting elements and measurement data be collected. In this case, an inverse matrix G⁻¹ is prepared in advance for each emitting element. The product of the inverse matrix G⁻¹ and vectors of the measurement data is calculated, and the scattered sound pressure intensity x is obtained a number of times equal to the number of emitting elements. The signal-to-noise ratio can be improved by combining the plurality of scattered sound pressure intensities x.

In a case where the inverse matrix G⁻¹ cannot be solved in the embodiment above, the scattered sound pressure intensity x can be obtained by solving the inverse matrix G⁻¹ using the method of least squares or regularization with a penalty term. In particular, in a case where the effect of noise n cannot be ignored, Gx + n = y and thus x = G⁻¹(y - n). This is because generally n cannot be specified.

### Simulation

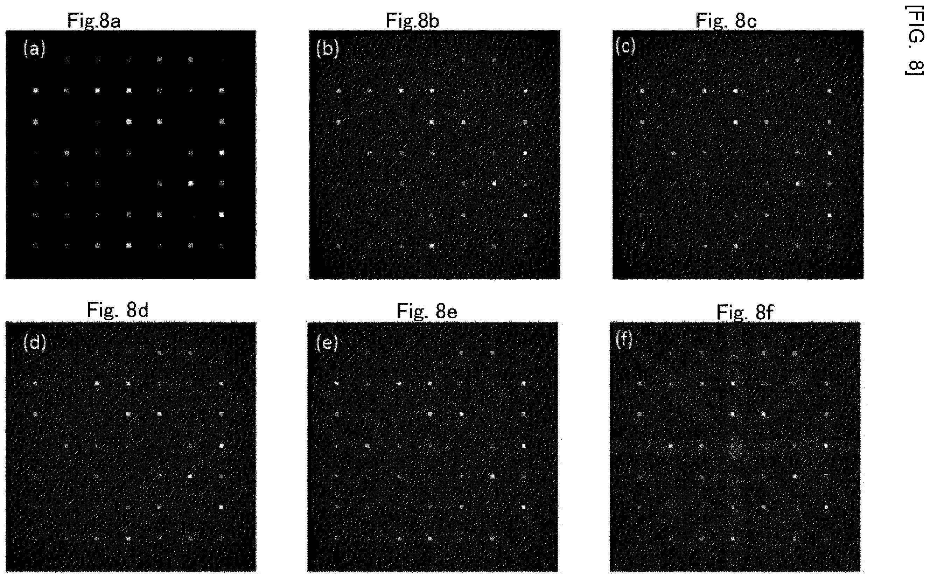

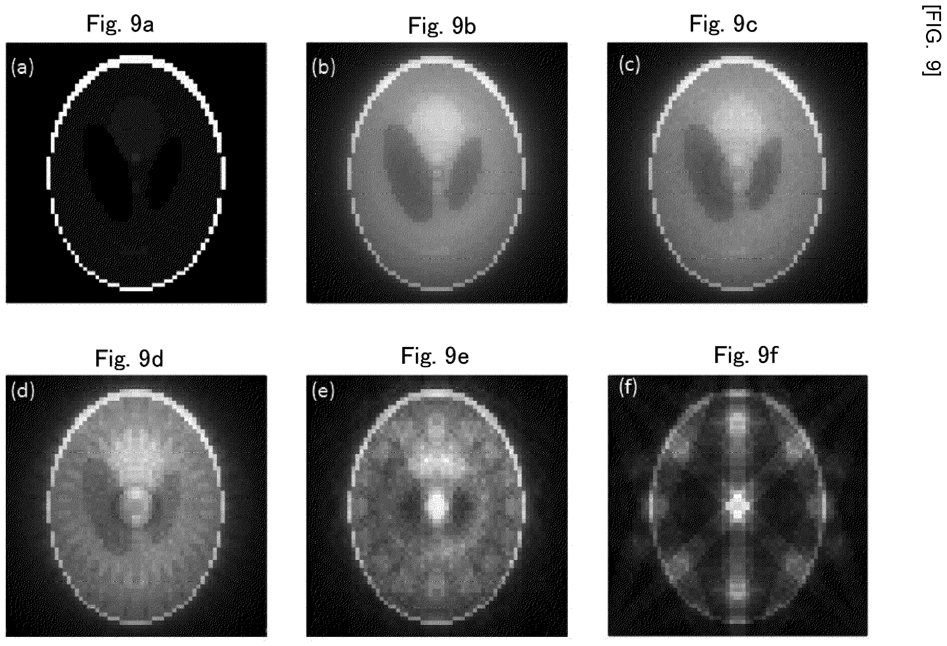

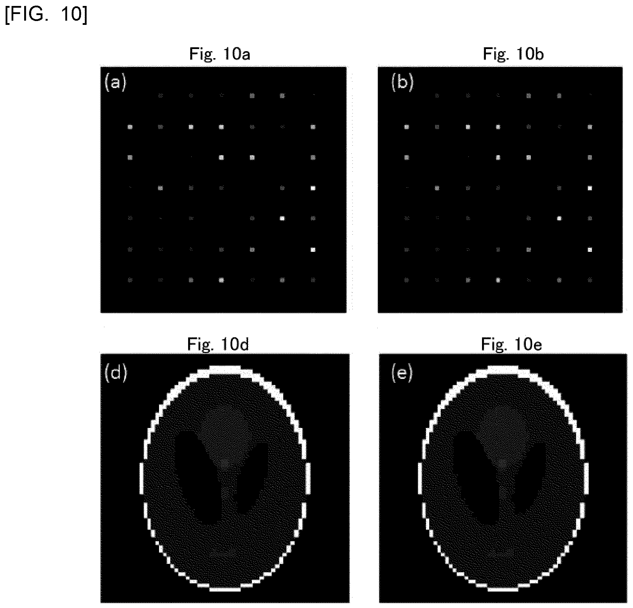

Simulations were run in which the scattering image generation method according to the above-described embodiment was applied to two kinds of phantoms. In addition, as a comparative example, a simulation was run in which the synthetic aperture method was applied. The phantoms are 49 (7 × 7) point scatterers arranged in a grid-like manner illustrated in Figs. 8a and 10a and the Shepp-Logan phantom with 64 × 64 pixels illustrated in Figs. 9a and 10d. Table 1 below illustrates the simulation conditions.

**[Table 1]**

| Number of Elements | 8 to 128 |
|---|---|
| Ring Array Radius | 50 mm |
| Sound Speed | 1500 m/s |
| Sampling Frequency | 5 MHz |
| Excitation | Unit Impulse |
| Region of Interest | 64 × 64 mm² |
| Pixel Size | 1 × 1 mm² |

Figs. 8 and 9 illustrate results obtained by applying the synthetic aperture method in the comparative example. Fig. 8 illustrates cases where 49 discrete scatterers were translated into models, and Fig. 9 illustrates cases where the configuration was translated into models. Figs. 8a and 9a illustrate the ground truth (the original models).

Figs. 8b to 8f and Figs. 9b to 9f respectively illustrate results of synthetic aperture imaging in cases where the number of emitting-receiving elements was 128, 64, 32, 16, and 8. As illustrated in Figs. 8 and 9, as the number of emitting-receiving elements decreases, it can be confirmed that noise occurs at pixels originally having no luminance. In particular, in Fig. 8, noise is only randomly distributed in the cases where the number of emitting-receiving elements is 128, 64, and 32; however, an artifact having a specific pattern is formed in the cases where the number of emitting-receiving elements is 16 and 8, which may result in for example a wrong diagnosis.

In Fig. 9, the contrast has decreased in the case where the number of emitting-receiving elements is 128 to the extent that isolation of a tumor from mammary glands is significantly difficult. In the images obtained in the cases where the number of emitting-receiving elements was 32, 16, and 8, the visibility of the images is significantly reduced due to occurrence of various artifacts.

In contrast, Fig. 10 illustrates results obtained by applying the method according to the present embodiment. Figs. 10a and 10d illustrate the ground truth (the original models), and Figs. 10b and 10e illustrate restored images obtained in a case where the number of emitting-receiving elements was eight. It is confirmed from Fig. 10 that the original models are perfectly restored.

In a case where the number of emitting elements was increased, the matrix G was formed by vertically arranging submatrices, the number of which is equal to the number of emission conditions. Furthermore, substantially the same results were confirmed also in a case where the number of emitting elements was 1 and the number of receiving elements was 16. Furthermore, reconstruction results similar to those of the case where the number of receiving elements was 16 were confirmed also in cases in which the number of emitting elements was 1 and the number of receiving elements was 32, 64, and 128.

From these results, two purposes can be confirmed, which are
[1] the number of emission conditions can be reduced to one while maintaining the number of elements constituting the ring array, and
[2] the number of elements constituting the ring array is reduced from 128 to 8, and only emission conditions, the number of which is equal to the number of elements, are imposed.

In the case of [1], compared with the synthetic aperture method, the imaging speed is 128 times faster and the amount of acquired data is 1/128. The number of elements is set to 128 in the example described above, and for example the imaging speed is 256 times faster and the amount of acquired data is 1/256 in a sequence under conditions in which 256 emissions are performed with 2048 elements.

In the case of [2], there are advantages in that cost such as the number of elements constituting the ring array, the circuit size of a multiplexer, and the number of cables between the elements and the multiplexer are reduced (1/16 in each case) and the size of the device is reduced.

The time required for imaging of a cross section is the diameter × 2/the sound speed × the number of imaging conditions. Thus, in a case where the diameter is 200 mm, the sound speed is 1500 m/s, and the number of imaging conditions for a classical synthetic aperture method is 256, it takes about 70 milliseconds to image a cross section. In a case where imaging of 1000 cross sections is performed, it takes about 70 seconds when the moving speed using a motor is sufficiently slow. When the method according to the present invention is applied, imaging of even 1000 cross sections takes 0.23 seconds as an imaging time. In this manner, the degree of freedom generated by increasing the speed of imaging and reducing the amount of data can be used to increase the number of images taken.

Regarding the amount of data, in the case of the synthetic aperture method in which the number of elements is 2048, the emission condition indicates 256, the ring has a diameter of 20 cm, the sampling frequency is 40 MHz, and an analog-to-digital (AD) converter produces outputs each consisting of 2 bytes, the number of sampling points in the depth direction (ultrasound wave propagation method) is 200e⁻³ × 2/1500 × 40e⁶ = 1e⁴, and each frame has 2 × 1e⁴ × 2048 × 256 = 10 GB. In a case where the number of cross sections is 1000, one volume data set needs 10 TB. Thus, it is required that the number of cross sections be reduced or image data (about 2 GB/volume) instead of echo data be stored. Better diagnosis capability can be expected by applying various types of application processing to one echo data set; however, it is unrealistic to store 10 TB data each time. In contrast, according to the present invention, the number of digits of data can be reduced roughly by two to three, and thus echo data itself can be stored, and for example data comparison with previous data becomes possible in the state of echo data before imaging, leading to the development of new uses for echo data.

A higher volume imaging speed makes it possible to greatly improve elastography, which is one of major applications of mammary gland imaging. Elastography is a technique for detecting a lesion by extracting a distortion using the cross-correlation between before and after addition of pressure on one line of a cross section and visualizing its distribution. The cross-correlation accuracy deceases when the line or slice is shifted due to addition of pressure. In general, a correlation error is suggested by fixing an imaging slice position and by causing the cross-correlation target to include only a line shift; however, if volume imaging is speeded up, the correlation error can be reduced because the correlation between echo lines in the volume can be used.

The description has been made so far on the assumption of an impulse response in the case of generation of the G matrix. In practice, the transducer is a band-pass filter with a resonant frequency at the center, and thus the bandwidth is finite. Even in this case, calculation is made possible by adding a waveform corresponding to an impulse response at the time of generation of the G matrix.

In addition, the effect caused by the heterogeneity of sound speed has not been discussed in the description made so far. That is, the sound speed of sound waves is nonuniform in a case where the sound waves pass through the inside of a breast and a case where the sound waves do not pass through the inside of the breast and also is nonuniform due to a change in the ratio of a portion of a single path going through the inside of the breast to a portion of the single path going through the outside of the breast. Thus, differences between the sound speeds of both the sound waves are preferably taken into consideration. For example, binarization processing for detecting the inside and outside of a breast is performed before a scattering image is generated, and the average sound speed in the breast can be calculated from an integral propagation time. Correction of the G matrix by using this sound speed distribution so as to cope with the heterogeneity of sound speed is effective in improving the robustness of the reconstruction algorithm.

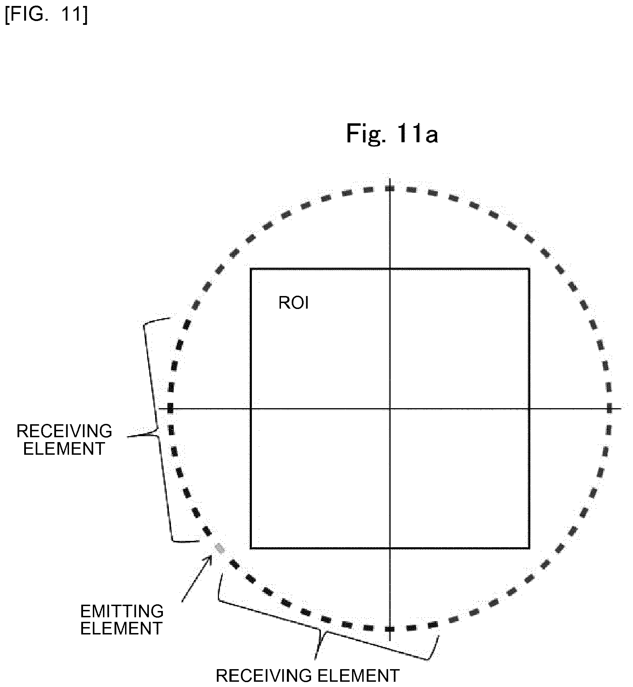

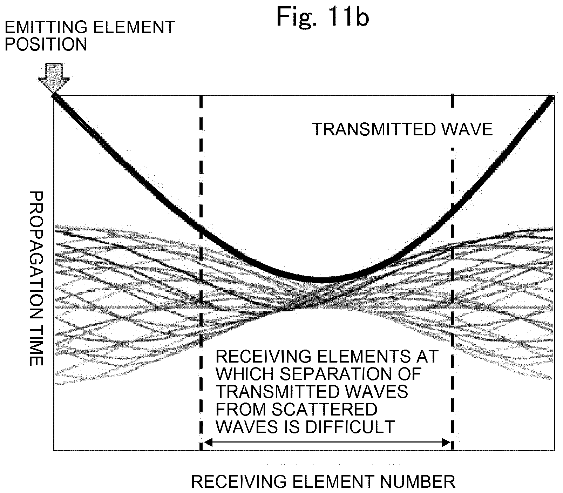

A point to note about generation of a G matrix is separation of transmitted waves. Although the strength of scattered waves and that of transmitted waves depend on the frequency used in emission, in general, transmitted waves are often stronger than scattered waves. As illustrated in Fig. 11b, when transmitted waves are superimposed on the scattered echo signals, reconfiguration using a method according to the present invention may be affected. Thus, as illustrated in Fig. 11a, the effect of the transmitted waves can be limited by imposing a receiving aperture restriction such that receiving is performed using only some elements arranged on the side where the emitting element is provided when viewed from a test object (imaging region). In the illustrated example, it is confirmed that the technique according to the present invention is feasible when a receiving aperture restriction is imposed such that the receiving aperture corresponds to 3/8 of all the elements.

In the embodiment described so far, the method has been described in which pixels are set at equal intervals in a wide region in the ring. When mammary gland tissue is imaged using a ring-shaped array, the imaging region is roughly divided into a region of the inside of the breast and a region of water surrounding the region. As a matter of course, the region of water does not need to be imaged for measurement. Thus, the number of pixels is reduced by setting the imaging region to only a region where a breast is present. Accordingly, the present invention makes it possible to reduce the number of necessary emission conditions and lower the sampling frequency. In a case where the ring array is close to the trunk of the body (the ring array is close to a top panel of the bed), the data reducing effect caused by not taking data of the water region is small; however, as the ring array moves away from the trunk of the body and approaches the tip of the breast, the cross-sectional area of the breast in the imaging plane is reduced, and the effect of reducing the amount of acquisition data becomes large under the imaging conditions optimized by the present invention. In this case, for example, when noise exists such as air bubbles present in the water, this noise may results in an error. Thus, regarding one condition, imaging is performed at different times and echo signals caused by scatterers floating in the water are removed by applying preprocessing in which only components that do not change over time are extracted, which is effective in reducing noise.

Next, conditions under which the present invention is realized and a method for setting major parameters will be supplementarily described. An advantageous point of the present invention is to perform discretization by dividing the measurement area into a grid and to handle the measurement area as a discrete model, which allows a matrix expression. In this case, the grid size is important. Compared with the classical conventional synthetic aperture method in which scattered waves are acquired and imaged, the way in which image quality changes with grid size will be described.

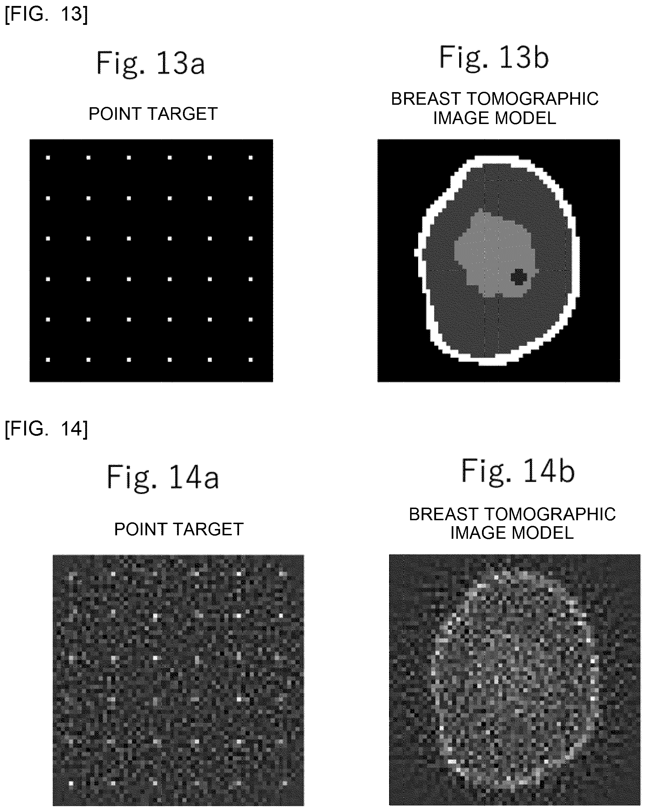

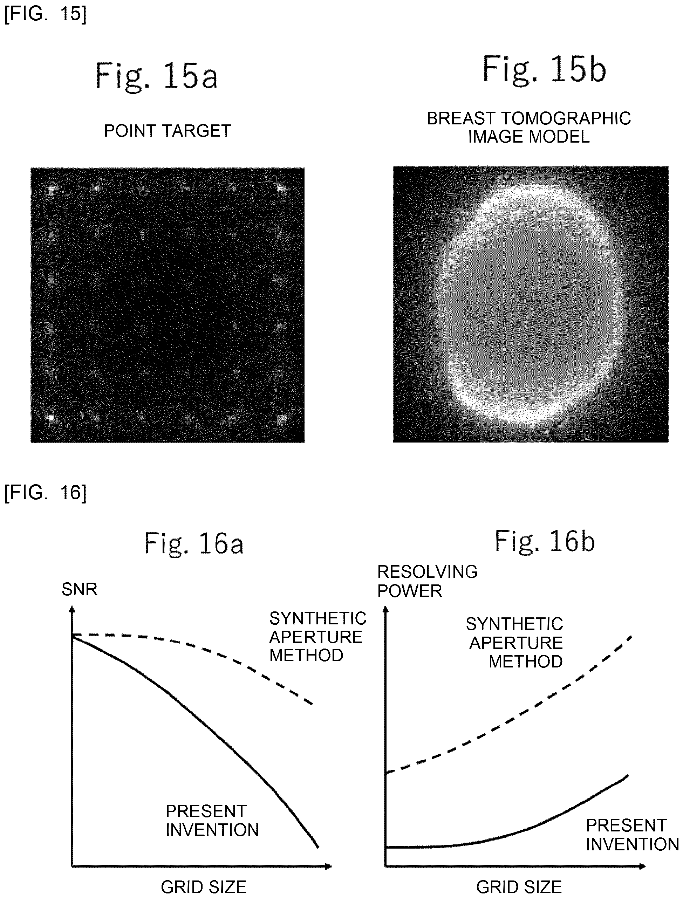

Fig. 13 illustrates two evaluation models. Fig. 13a illustrates the configuration of an array of points, and Fig. 13b illustrates a simulated configuration of a breast tomographic image. When the center frequency is set to 2 MHz and the space is divided into a grid with a pixel size of 0.2 mm (about one eighth of the wavelength), the total sum of the amounts of scattering of scatterers present in each grid quadrangle is treated to be present at the center of the grid quadrangle. In this case, an approximation operation for replacing the spatial distribution of the scatterers in the grid quadrangle with the δ function present at the center of the grid quadrangle may cause generation of artifacts in the process of image reconstruction and a reduction in resolution. As typical results, Figs. 14a and 14b illustrate results according to the present invention, and Figs. 15a and 15b illustrate results obtained when the known synthetic aperture method was used as a comparative example. In Figs. 14a and 14b, the resolution is maintained; however, noise has been significantly increased. In contrast, in Figs. 15a and 15b, noise has not been significantly increased; however, the resolution has been significantly decreased.

This is why two image quality evaluation factors were analyzed with the horizontal axis representing scatterer position error in each grid quadrangle (the distance between the set position and the center of the grid quadrangle). That is, regarding a case where the synthetic aperture method was used and a case of the present invention, plots were placed in Figs. 16a and 16b with the vertical axis representing signal-to-noise ratio (SNR) and resolution (a half width). Consequently, in the case of the synthetic aperture method, as the error increases, the resolution decreases (the resolving power = resolvable lower limit size [m] increases); however, there is no large change in the SNR. In contrast, in the case of the present invention, the change in the resolution is small; however, the change in the SNR is large.

A reduction in the resolution causes blurring of the image but does not cause an observation target to disappear. (As a matter of course, whether the observation target disappears depends on how much the resolution is reduced.) In contrast, when the SNR goes below a certain value, the observation target cannot be visually identified at all. When consideration is given within a range in which calculation of the inverse matrix is practically possible, the grid size becomes large, the position error increases, and the SNR rapidly decreases. Thus, a technique such as the present invention has not been considered hitherto. The present invention has been made from the idea that a target can be handled in a discrete manner if there are a sufficient number of grid quadrangles. As these results show, the present invention requires stricter conditions to work effectively compared to the synthetic aperture method; however, under those conditions, the present invention can realize higher performance.

In addition, as another embodiment, an example will be described in which the present invention is used to measure a change in the temperature inside the breast. Cancer cells have a faster proliferation rate than cells constituting other normal tissues and are known to have active metabolism in order to achieve quick proliferation. Thus, for the existing positron emission tomography (PET), a diagnostic method in which a site with active metabolism = a tumor is detected by giving sugar labeled with an isotope that decays radioactively and by visualizing the spatial distribution of a source of y rays emitted from the site with active metabolism is widely used in clinical settings. This technique can detect lesions with high contrast, but the facts that incidental facilities such as an accelerator for generating a radioisotope drug are expensive and large and that internal exposure occurs interfere with the utilization of this technique in medical examinations.

As a simpler metabolic measurement method, a technique for detecting a site of temperature rise due to metabolism using an infrared camera has a long history of study and clinical equipment using the method was once sold. (As artificial intelligence (AI) technology develops, some groups have recently been considering redevelopment of such equipment.) In a case where measurement is performed using infrared rays, there is a problem in that a measurable area is limited to a site at shallow depths close to the body surface since moisture in the living body absorbs infrared rays caused by thermal radiation.

In measurement using a ring array, it is possible to measure a change in the distribution of sound speed caused by a change in temperature. In actuality, the temperature dependence of sound speed in moisture or fat is on the order of X m/s/k and can be detected even by a ring array. This measurement has an advantage in that a site at deep depths in the body is theoretically measurable, compared with the case using an infrared camera. Note that what can be measured from the temperature dependence of sound speed is not the absolute temperature but a change in temperature, and thus this measurement is effective in acquiring contrast from the difference between the temperature change rate of a site having a high metabolic rate and that of another site after the temperature of the breast is caused to change. In a normal ring-echo imaging sequence, which is not based on the present invention, it takes five to ten minutes to acquire a volume data set. Heat generated at a tumor diffuses according to the heat diffusion equation, and the temperature difference between the heat source and the surrounding tissue other than the heat source is small in a state of equilibrium. To efficiently acquire temperature changes until this state of equilibrium is reached and to cause the temperature of a breast of an examinee to change without provision of additional equipment, it is desirable to observe the nonequilibrium process of temperature right after the examinee inserts her breast into a water tank in which a ring array is stored.

For this purpose, according to the present invention, a reduction in the number of emission conditions is effective. With respect to an acquisition time of five minutes for the one volume data set described above, the transient temperature change time is about five to ten minutes. To observe transient temperature changes during this period, the imaging speed needs to be five to ten times faster. Scattering images are successively generated every predetermined time using the acceleration technique based on the present invention, a region having a large change (a portion having pixel values or corresponding RF data that have changed by an amount greater than or equal to a predetermined value) is extracted from image changes over time, and it is converted into the magnitude of temperature change. Consequently, it becomes possible to visualize differences in thermal resistance due to metabolism.

### Reference Signs List

- 10: ultrasound diagnostic system
- 110: switch circuit
- 120: emission-reception circuit
- 130: calculation device
- 140: image display device

## Claims

1. An ultrasound diagnostic system comprising:
a plurality of elements that are arranged around a test object and perform at least either emission or reception of ultrasound;
a control unit that controls the plurality of elements such that at least one of the plurality of elements emits ultrasound and all or some of the plurality of elements receive scattered waves caused by the test object scattering the ultrasound;
a data collection unit that collects measurement data, which are data obtained from the elements that have received the scattered waves;
a calculation unit that calculates, for division regions into which an imaging region including all or a portion of the test object is divided, a scattered sound pressure intensity of each division region, which is the intensity of sound pressure of the scattered waves in the division region, on the basis of a first factor and a second factor of the division region, the first factor being constituted by arrival times which are each a period from emission to reception of ultrasound that is emitted from a predetermined element among the plurality of elements, scattered by the test object in the division region, and received by a corresponding one of all or some of the plurality of elements, the second factor being constituted by the measurement data; and
an image generation unit that generates a scattering image, which is an image obtained by converting the scattered sound pressure intensity of each division region into a pixel value.

2. The ultrasound diagnostic system according to Claim 1, wherein the division regions are regions obtained by dividing the imaging region in a grid-like manner,
the first factor is an inverse matrix of a matrix constituted by the arrival times,
the second factor is a vector constituted by the measurement data, and
the calculation unit calculates the scattered sound pressure intensity from the product of the first factor and the second factor.

3. The ultrasound diagnostic system according to Claim 2, wherein the product of the number of vertical regions and the number of horizontal regions, the vertical and horizontal regions constituting the division regions and being obtained by performing division in a grid-like manner, and the product of the number of receiving elements and the number of data samples in a time-axis direction in collection of the measurement data respectively match the number of columns and the number of rows of the matrix.

4. The ultrasound diagnostic system according to Claim 2 or 3, wherein the control unit performs control such that a second element emits ultrasound after a first element emits ultrasound,
the data collection unit collects first measurement data from an element that has received a scattered wave corresponding to the ultrasound emitted by the first element and collects second measurement data from an element that has received a scattered wave corresponding to the ultrasound emitted by the second element, and
the calculation unit calculates a first scattered sound pressure intensity from the product of a first inverse matrix obtained in a case where the first element is treated as an ultrasound emitting element and a vector obtained by arranging the first measurement data, calculates a second scattered sound pressure intensity from the product of a second inverse matrix obtained in a case where the second element is treated as an ultrasound emitting element and a vector obtained by arranging the second measurement data, and combines the first scattered sound pressure intensity and the second scattered sound pressure intensity.

5. The ultrasound diagnostic system according to Claim 3, wherein a rank of the matrix is equal to the product of the number of vertical regions and the number of horizontal regions, the vertical and horizontal regions being regions obtained by performing division in the grid-like manner.

6. The ultrasound diagnostic system according to Claim 5, wherein the arrival times are calculated on the basis of the fact that there is a difference in the sound speed of the ultrasound inside a breast and the sound speed of the ultrasound outside the breast.

7. The ultrasound diagnostic system according to Claim 5, wherein when seen from the test object, the receiving elements are arranged on a side where the emitting element is arranged.

8. The ultrasound diagnostic system according to Claim 1, wherein the scattering image is generated every predetermined time and a portion of the generated scattering image where a change in pixel value is greater than or equal to a predetermined value is extracted.

9. An ultrasound diagnostic method comprising:
a step of emitting ultrasound from any one of a plurality of elements arranged around a test object and receiving, using all or some of the plurality of elements, scattered waves caused by the test object scattering the ultrasound;
a step of collecting measurement data, which are data obtained from the elements that have received the scattered waves;
a step of calculating, for division regions into which an imaging region including all or a portion of the test object is divided, a scattered sound pressure intensity of each division region, which is the intensity of sound pressure of the scattered waves in the division region, on the basis of a first factor and a second factor of the division region, the first factor being constituted by arrival times which are each a period from emission to reception of ultrasound that is emitted from a predetermined element among the plurality of elements, scattered by the test object in the division region, and received by a corresponding one of all or some of the plurality of elements, the second factor being constituted by the measurement data; and
a step of generating a scattering image, which is an image obtained by converting the scattered sound pressure intensity of each division region into a pixel value.

## Patentansprüche

1. Ultraschalldiagnosesystem, umfassend:
eine Vielzahl von Elementen, die um ein Testobjekt herum angeordnet ist und mindestens entweder eine Emission oder einen Empfang von Ultraschall durchführt;
eine Steuereinheit, welche die Vielzahl von Elementen derart steuert, dass mindestens eine der Vielzahl von Elementen Ultraschall emittiert und alle oder einige von der Vielzahl von Elementen Streuwellen empfangen, die von dem Testobjekt, das den Ultraschall streut, verursacht werden;
eine Datensammeleinheit, die gemessene Daten sammelt, die Daten sind, die von den Elementen, welche die Streuwellen empfangen haben, erhalten werden;
eine Recheneinheit, die für Teilungsregionen, in die eine Bildgebungsregion der gesamten oder eines Teils des Testobjekts aufgeteilt wird, eine gestreute Schalldruckintensität von jeder Teilungsregion, welche die Intensität von Schalldruck der Streuwellen in der Teilungsregion ist, auf der Basis eines ersten Faktors und eines zweiten Faktors der Teilungsregion berechnet, wobei der erste Faktor aus Eintreffzeiten besteht, die jeweils ein Zeitraum von einer Emission bis zum Empfang von Ultraschall darstellen, der von einem vorbestimmten Element unter der Vielzahl von Elementen emittiert wird, der von dem Testobjekt in der Teilungsregion gestreut wird und von einem Entsprechenden von allen oder einigen der Vielzahl von Elementen empfangen wird, wobei der zweite Faktor aus den Messdaten besteht; und
eine Bilderzeugungseinheit, die ein gestreutes Bild erzeugt, das ein Bild ist, das durch Umwandeln der gestreuten Schalldruckintensität von jeder Teilungsregion in einen Pixelwert erhalten wird.

2. Ultraschalldiagnosesystem nach Anspruch 1, wobei die Teilungsregionen Regionen sind, die durch Aufteilen der Bildgebungsregion auf eine gitterähnliche Weise erhalten werden,
der erste Faktor eine inverse Matrix einer Matrix ist, die aus den Eintreffzeiten besteht,
der zweite Faktor ein Vektor ist, der aus den Messdaten besteht, und
die Recheneinheit die gestreute Schalldruckintensität aus dem Produkt des ersten Faktors und des zweiten Faktors berechnet.

3. Ultraschalldiagnosesystem nach Anspruch 2, wobei das Produkt der Anzahl von vertikalen Regionen und der Anzahl von horizontalen Regionen, wobei die vertikalen und horizontalen Regionen aus den Teilungsregionen bestehen und durch Durchführen einer Aufteilung auf eine gitterähnliche Weise erhalten werden, und das Produkt der Anzahl von empfangenden Elementen und der Anzahl von Datenmustern in einer Zeitachsenrichtung bei dem Sammeln von Messdaten jeweils der Anzahl von Spalten und der Anzahl von Reihen der Matrix entsprechen.

4. Ultraschalldiagnosesystem nach Anspruch 2 oder 3, wobei die Steuereinheit eine Steuerung durchführt, sodass ein zweites Element Ultraschall emittiert, nachdem ein erste Element Ultraschall emittiert hat,
die Datenerfassungseinheit erste Messdaten von einem Element sammelt, das eine Streuwelle empfangen hat, die dem Ultraschall entspricht, der von dem ersten Element emittiert wurde, und zweite Messdaten von einem Element sammelt, das eine Streuwelle empfangen hat, die dem Ultraschall entspricht, der von dem zweiten Element emittiert wurde, und
die Recheneinheit eine erste gestreute Schalldruckintensität aus dem Produkt einer ersten inversen Matrix, die in einem Fall erhalten wurde, in dem das erste Element als ein Ultraschall emittierendes Element behandelt wird, und einem Vektor berechnet, der durch Anordnen der ersten Messdaten erhalten wird, eine zweite gestreute Schalldruckintensität aus dem Produkt einer zweiten inversen Matrix, die in einem Fall erhalten wird, in dem das zweite Element als ein Ultraschall emittierendes Element behandelt wird, und einem Vektor berechnet, der durch Anordnen der zweiten Messdaten erhalten wird, und die erste gestreute Druckschallintensität mit der zweiten gestreuten Druckschallintensität kombiniert.

5. Ultraschalldiagnosesystem nach Anspruch 3, wobei eine Rang der Matrix gleichwertig dem Produkt der Anzahl von vertikalen Regionen und der Anzahl von horizontalen Regionen ist, wobei die vertikalen und horizontalen Regionen Regionen sind, die durch Durchführen einer Aufteilung auf eine gitterähnliche Weise erhalten werden.

6. Ultraschalldiagnosesystem nach Anspruch 5, wobei die Eintreffzeiten auf der Basis der Tatsache berechnet werden, dass eine Differenz der Schallgeschwindigkeit des Ultraschalls im Inneren einer Brust und der Schallgeschwindigkeit des Ultraschalls außerhalb der Brust besteht.

7. Ultraschalldiagnosesystem nach Anspruch 5, wobei, wenn aus der Sicht des Testobjekts betrachtet, die empfangenden Elemente an einer Seite angeordnet sind, an der das emittierende Element angeordnet ist.

8. Ultraschalldiagnosesystem nach Anspruch 1, wobei das streuende Bild an jedem vorbestimmten Zeitpunkt erzeugt wird und ein Teil des erzeugten streuenden Bilds, in dem eine Veränderung des Pixelwerts höher als oder gleichwertig einem vorbestimmten Wert ist, extrahiert wird.

9. Ultraschalldiagnoseverfahren, umfassend:
einen Schritt des Emittierens von Ultraschall von einem beliebigen einer Vielzahl von Elementen, die um ein Testobjekt herum angeordnet sind und Empfangen, unter Verwendung aller oder einiger der Vielzahl von Elementen, von Streuwellen, die von dem Testobjekt, das den Ultraschall streut, verursacht werden;
einen Schritt des Sammelns von Messdaten, die Daten sind, die von den Elementen erhalten werden, welche die Streuwellen empfangen haben;
einen Schritt des Berechnens für Teilungsregionen, in die eine Bildgebungsregion der gesamten oder eines Teils des Testobjekts aufgeteilt wird, einer gestreuten Schalldruckintensität von jeder Teilungsregion, welche die Intensität von Schalldruck der Streuwellen in der Teilungsregion ist, auf der Basis eines ersten Faktors und eines zweiten Faktors der Teilungsregion, wobei der erste Faktor aus Eintreffzeiten besteht, die jeweils ein Zeitraum von einer Emission bis zum Empfang von Ultraschall darstellen, der von einem vorbestimmten Element unter der Vielzahl von Elementen emittiert wird, der von dem Testobjekt in der Teilungsregion gestreut wird und von einem Entsprechenden von allen oder einigen der Vielzahl von Elementen empfangen wird, wobei der zweite Faktor aus den Messdaten besteht; und
einen Schritt des Erzeugens eines gestreuten Bilds, das ein Bild ist, das durch Umwandeln der gestreuten Schalldruckintensität von jeder Teilungsregion in einen Pixelwert erhalten wird.

## Revendications

1. Système de diagnostic par ultrasons comprenant :
une pluralité d'éléments qui sont agencés autour d'un objet de test et effectuent au moins une émission ou une réception d'ultrasons ;
une unité de commande qui commande la pluralité d'éléments de telle sorte qu'au moins un de la pluralité d'éléments émet des ultrasons et la totalité ou une partie de la pluralité d'éléments reçoivent des ondes diffusées causées par l'objet de test diffusant les ultrasons ;
une unité de collecte de données qui collecte des données de mesure, qui sont des données obtenues à partir des éléments qui ont reçu les ondes diffusées ;
une unité de calcul qui calcule, pour des régions de division dans lesquelles est divisée une région d'imagerie incluant la totalité ou une partie de l'objet de test, une intensité de pression sonore diffusée de chaque région de division, qui est l'intensité de pression sonore des ondes diffusées dans la région de division, sur la base d'un premier facteur et d'un second facteur de la région de division, le premier facteur étant constitué par des temps d'arrivée qui sont chacun une période allant de l'émission à la réception d'ultrasons qui sont émis depuis un élément prédéterminé parmi la pluralité d'éléments, diffusés par l'objet de test dans la région de division, et reçus par un élément correspondant de la totalité ou d'une partie de la pluralité d'éléments, le second facteur étant constitué par les données de mesure ; et
une unité de génération d'image qui génère une image de diffusion, qui est une image obtenue en convertissant l'intensité de pression sonore diffusée de chaque région de division en une valeur de pixel.

2. Système de diagnostic par ultrasons selon la revendication 1, dans lequel les régions de division sont des régions obtenues en divisant la région d'imagerie à la manière d'une grille,
le premier facteur est une matrice inverse d'une matrice constituée par les temps d'arrivée,
le second facteur est un vecteur constitué par les données de mesure, et
l'unité de calcul calcule l'intensité de pression sonore diffusée à partir du produit du premier facteur et du second facteur.

3. Système de diagnostic par ultrasons selon la revendication 2, dans lequel le produit du nombre de régions verticales et du nombre de régions horizontales, les régions verticales et horizontales constituant les régions de division et étant obtenues en effectuant une division à la manière d'une grille, et le produit du nombre d'éléments de réception et du nombre d'échantillons de données dans une direction d'axe de temps lors de la collecte des données de mesure correspondent respectivement au nombre de colonnes et au nombre de rangées de la matrice.

4. Système de diagnostic par ultrasons selon la revendication 2 ou 3, dans lequel l'unité de commande effectue une commande de telle sorte qu'un second élément émet des ultrasons après qu'un premier élément a émis des ultrasons,
l'unité de collecte de données collecte des premières données de mesure à partir d'un élément qui a reçu une onde diffusée correspondant aux ultrasons émis par le premier élément et collecte des secondes données de mesure à partir d'un élément qui a reçu une onde diffusée correspondant aux ultrasons émis par le second élément, et
l'unité de calcul calcule une première intensité de pression sonore diffusée à partir du produit d'une première matrice inverse obtenue dans un cas où le premier élément est traité comme élément d'émission d'ultrasons et d'un vecteur obtenu en agençant les premières données de mesure, calcule une seconde intensité de pression sonore diffusée à partir du produit d'une seconde matrice inverse obtenue dans un cas où le second élément est traité comme élément d'émission d'ultrasons et d'un vecteur obtenu en agençant les secondes données de mesure, et combine la première intensité de pression sonore diffusée et la seconde intensité de pression sonore diffusée.

5. Système de diagnostic par ultrasons selon la revendication 3, dans lequel un rang de la matrice est égal au produit du nombre de régions verticales et du nombre de régions horizontales, les régions verticales et horizontales étant des régions obtenues en effectuant une division à la manière d'une grille.

6. Système de diagnostic par ultrasons selon la revendication 5, dans lequel les temps d'arrivée sont calculés sur la base du fait qu'il y a une différence de la vitesse sonore des ultrasons à l'intérieur d'une poitrine et de la vitesse sonore des ultrasons à l'extérieur de la poitrine.

7. Système de diagnostic par ultrasons selon la revendication 5, dans lequel, lorsque l'on regarde depuis l'objet de test, les éléments de réception sont agencés sur un côté où est agencé l'élément d'émission.

8. Système de diagnostic par ultrasons selon la revendication 1, dans lequel l'image de diffusion est générée chaque temps prédéterminé et une partie de l'image de diffusion générée où un changement de valeur de pixel est supérieur ou égal à une valeur prédéterminée est extraite.

9. Procédé de diagnostic par ultrasons comprenant :
une étape d'émission d'ultrasons à partir de l'un quelconque d'une pluralité d'éléments agencés autour d'un objet de test et de réception, à l'aide de la totalité ou d'une partie de la pluralité d'éléments, d'ondes diffusées causées par l'objet de test diffusant les ultrasons ;
une étape de collecte de données de mesure, qui sont des données obtenues à partir des éléments qui ont reçu les ondes diffusées ;
une étape de calcul, pour des régions de division dans lesquelles est divisée une région d'imagerie incluant la totalité ou une partie de l'objet de test, d'une intensité de pression sonore diffusée de chaque région de division, qui est l'intensité de pression sonore des ondes diffusées dans la région de division, sur la base d'un premier facteur et d'un second facteur de la région de division, le premier facteur étant constitué par des temps d'arrivée qui sont chacun une période allant de l'émission à la réception d'ultrasons qui sont émis depuis un élément prédéterminé parmi la pluralité d'éléments, diffusés par l'objet de test dans la région de division, et reçus par un élément correspondant de la totalité ou d'une partie de la pluralité d'éléments, le second facteur étant constitué par les données de mesure ; et
une étape de génération d'une image de diffusion, qui est une image obtenue en convertissant l'intensité de pression sonore diffusée de chaque région de division en une valeur de pixel.
